Europäisches Patentamt

European Patent Office

Office européen des brevets

(11) Veröffentlichungsnummer: **0 376 088**
**A1**

(12) **EUROPÄISCHE PATENTANMELDUNG**

(21) Anmeldenummer: 89123217.5

(22) Anmeldetag: 15.12.89

(51) Int. Cl.5: **C07C 69/54, C07C 67/08**

(30) Priorität: 24.12.88 DE 3843854

(43) Veröffentlichungstag der Anmeldung:
04.07.90 Patentblatt 90/27

(84) Benannte Vertragsstaaten:
GR

(71) Anmelder: Henkel Kommanditgesellschaft auf Aktien
Postfach 1100 Henkelstrasse 67
D-4000 Düsseldorf-Holthausen(DE)

(72) Erfinder: Ritter, Wolfgang, Dr.
Am Bandenfeld 74
D-5657 Haan(DE)
Erfinder: Sitz, Hans-Dieter, Dr.
Widdeshovener Strasse 48
D-4049 Rommerskirchen(DE)
Erfinder: Speitkamp, Ludwig
Tönisstrasse 13
D-4000 Düsseldorf 13(DE)

(54) Verfahren zur verbesserten Herstellung von (Meth)acrylsäureestern mehrwertiger Alkohole (I).

(57) Beschrieben wird ein Verfahren zur Herstellung von (Meth)acrylsäureestern mehrwertiger Alkohole durch Umsetzung der Reaktanten in Gegenwart von sauren Veresterungskatalysatoren unter Zusatz von alpha-substituierten phenolischen Verbindungen, insbesondere Di-tert.-butylhydrochinon, als Polymerisationsinhibitoren. Das Kennzeichen des neuen Verfahrens liegt darin, daß man mit bei Reaktionstemperatur flüssigen Reaktionsgemischen arbeitet, die wenigstens weitgehend frei von Lösungs- und/oder azeotropen Schleppmitteln sind und dabei das entstehende Kondensationswasser aus der Gasphase des Reaktionsraumes abzieht.

EP 0 376 088 A1

Verfahren zur verbesserten Herstellung von (Meth)acrylsäureestern mehrwertiger Alkohole (I)

Die Erfindung betrifft ein Verfahren zur Herstellung von polyfunktionellen Estern der Acrylsäure und/oder der Methacrylsäure mit mehrwertigen Alkoholen - im folgenden auch als (Meth)acrylsäureester bzw. Poly(Meth)acrylsäureester bezeichnet - durch Umsetzung der Reaktanten in Gegenwart saurer Veresterungskatalysatoren unter Zusatz von Polymerisationsinhibitoren zum Reaktionsgemisch.

(Meth)acrylsäureester mehrwertiger Alkohole insbesondere aus der Gruppe der 2- bis 4wertigen aliphatischen gesättigten Alkohole und deren Oxalkylierungsprodukte finden zunehmende Bedeutung als hochreaktive Bestandteile in strahlenhärtenden Systemen. Solche polyfunktionellen (Meth)acrylsäureester können beispielsweise als Lackrohstoffe für die Elektronenstrahlhärtung oder als Bestandteil von UV-Licht-härtenden Druckfarben oder entsprechenden Überzugslacken, Spachtel, Form- oder Vergußmassen sowie in Klebstoffen, insbesondere anaerob härtenden Klebstoffen Verwendung finden. Ihre Herstellung ist allerdings nicht problemlos. Gefordert werden insbesondere farblose Produkte mit geringer Säurezahl und hoher Lagerstabilität, die praktisch auch keinen Eigengeruch aufweisen. Eine destillative Reinigung der (Meth)acrylsäureester der hier betroffenen Art scheidet in aller Regel aufgrund ihres hohen Molekulargewichtes als möglichst farblose Reaktionsprodukte der Veresterung anfallen. Die Durchführung der Veresterungsreaktion fordert die Mitverwendung hochwirksamer Inhibitoren, die ihrerseits keine unerwünschten Nebenreaktionen, beispielsweise Verfärbungen, auslösen.

Zur Herstellung solcher polyfunktioneller (Meth)acrylsäureester mehrwertiger Alkohole besteht umfangreiche Vorliteratur. Verwiesen sei insbesondere auf die deutsche Offenlegungsschrift 29 13 218 und die darin zitierte einschlägige Literatur. So ist es aus der DE-AS 12 67 547 und aus der Zeitschrift "Chem. and Ind." 18 (1970), 597, bekannt, polyfunktionelle (Meth)acrylsäureester durch azeotrope Veresterung der (Meth)acrylsäure mit mehrwertigen Alkoholen in Gegenwart von azeotropen Schleppmitteln sowie von sauren Katalysatoren und Polymerisationsinhibitoren herzustellen. Als Polymerisationsinhibitoren wurden vorgeschlagen Phenole, Phenolderivate, Kupfer, Kupferverbindungen oder Phenothiazin. Als saure Katalysatoren werden organische oder anorganische Säuren oder saure Ionenaustauscher eingesetzt, wobei p-Toluolsulfonsäure und Schwefelsäure bevorzugt sein können. Die Veresterung erfolgt insbesondere bei Temperaturen von 40 bis 120 °C. Geeignete azeotrope Schleppmittel für die Entfernung des Reaktionswassers sind aliphatische oder cycloaliphatische oder aromatische Kohlenwasserstoffe bzw. deren Gemische mit Siedebereichen innerhalb der angegebenen Temperaturgrenzen.

In der genannten DE-OS 29 13 218 wird vorgeschlagen, die azeotrope Veresterung in Gegenwart mindestens eines organischen Esters der phosphorigen Säure zusätzlich zu einem mitverwendeten Inhibitor auf Phenolbasis durchzuführen. Zwingend wird allerdings auch hier die Mitverwendung mindestens eines aliphatische, cycloaliphatischen und/oder aromatischen Kohlenwasserstoffs mit einem Siedepunkt im Bereich von 40 bis 120 °C gefordert. Das entstehende Reaktionswasser soll mittels dieser Schleppmittel azeotrop ausgekreist werden. Die Reaktionsdauer wird nach den Beispielen dieser Druckschrift mit 10 bis 18 Stunden angesetzt.

Die Erfindung geht von der Aufgabe aus, Reaktionsbedingungen für die hier betroffene Veresterungsreaktion zu ermitteln, die einerseits zu einer substantiellen Abkürzung der Reaktionsdauer führen können, andererseits aber die Qualität der entstehenden Veresterungsprodukte und insbesondere die hohe Farbqualität nicht negativ beeinflussen. Die Erfindung will weiterhin darauf verzichten können, vergleichsweise komplexe Inhibitorsysteme derart einsetzen zu müssen, wie sie in der genannten DE-OS 29 13 218 beschrieben sind. Es soll dabei erfindungsgemäß auch möglich sein, den im praktischen Einsatz gewünschten Applikationsinhibitor der hier betroffenen hochreaktiven Systeme gleichzeitig schon als Reaktionsinhibitor bei der Synthese der polyfunktionellen (Meth)acrylsäureester einzusetzen.

Die technische Lösung der erfindungsgemäßen Aufgabenstellungen geht von der Erkenntnis aus, daß vergleichsweise hochreine Veresterungsprodukte als unmittelbare Verfahrensprodukte selbst dann erhalten werden können, wenn auf die Mitverwendung von Verdünnungsmitteln bzw. azeotropen Schleppmitteln verzichtet wird und daß es unter den Bedingungen des lösungsmittelfreien Arbeitens sogar möglich ist, vergleichsweise schärfere Veresterungsbedingungen einzusetzen, die zu einer beträchtlichen Abkürzung der Reaktionszeit führen. Voraussetzung hierfür ist insbesondere, daß der richtige Polymerisationsinhibitor gewählt und unter den nachfolgenden geschilderten Verfahrensbedingungen gearbeitet wird.

Gegenstand der Erfindung ist dementsprechend ein Verfahren zur Herstellung von (Meth)acrylsäureestern mehrwertiger Alkohole durch deren Umsetzung mit Acrylsäure und/oder Methacrylsäure in Gegenwart von sauren Veresterungskatalysatoren unter Zusatz von alpha-substituierten phenolischen Verbindungen als Polymerisationsinhibitoren. Das neue Verfahren ist dadurch gekennzeichnet, daß man mit bei Reaktionstemperatur flüssigen Re aktionsgemischen arbeitet, die wenigstens weitgehend frei von Lösungs- und/oder azeotropen Schleppmitteln sind und daß man das entstehende Kondensationswasser aus der Gasphase des Reaktionsraumes abzieht. Als bevorzugter Polymerisationsinhibitor aus der Klasse der alpha-substituierten phenolischen Verbindungen wird das 2,5-Di-tert.-butylhydrochinon eingesetzt. Bevorzugt wird ohne jeden Zusatz von Lösungs- und/oder azeotropen Schleppmitteln gearbeitet.

Es ist erfindungsgemäß weiterhin bevorzugt, den Reaktionsraum mit einem Gasstrom zu durchspülen und diesen Gasstrom insbesondere dazu zu benutzen, das Kondensationswasser aus der Veresterungsreaktion aus dem Reaktor auszuschleusen. Bevorzugt wird dabei mit einem Gasstrom gearbeitet, der einen beschränkten Anteil an freiem Sauerstoff enthält. In Abhängigkeit von den jeweils gewählten Verfahrensbedingungen kann Luft oder ein an Sauerstoff verarmtes Gasgemisch eingesetzt werden. Ein Beispiel der zuletzt genannten Art sind Stickstoff/Luft-Gemische. In aller Regel wird allerdings ein gewisser Gehalt an freiem Sauerstoff in dieser der Reaktionsmischung zugeführten Gasphase gewünscht. Diese beschränkten Sauerstoffmengen aktivieren in an sich bekannter Weise den Inhibitor während des Reaktionsgeschehens.

Der Sauerstoffgehalt des Gasgemisches liegt im allgemeinen bei wenigstens etwa 1 Volumen-% und bevorzugt im Bereich von etwa 2 bis 20 Volumen-%. Aus Gründen der Reaktionssicherheit kann es dabei bevorzugt sein, mit Gehalten an freiem Sauerstoff in der unteren Hälfte des genannten Bereiches, also bis etwa 10 Volumen-% und bevorzugt bis etwa 7 Volumen-% zu arbeiten. Der Gasstrom wird in einer bevorzugten Ausführungsform der Erfindung in das flüssige Reaktionsgemisch eingespeist und kann dieses beispielsweise feinverteilt durchperlen. Es wird dabei zweckmäßigerweise mit begrenzten Mengen dieses Gasstromes gearbeitet, so daß kein unerwünscht hoher Austrag an Reaktions komponenten - insbesondere der vergleichweise leichter flüchtigen Säuren - stattfindet.

Als Polymerisationsinhibitoren können bestimmt ausgewählte einzelne Verbindungen als auch mehrere Komponenten aus der Klasse der alpha-substituierten Phenolverbindungen eingesetzt werden. Bevorzugt sind vergleichsweise schwer flüchtige Verbindungen, insbesondere auf Basis entsprechend substituierter einwertiger oder mehrwertiger Phenole, wobei als mehrwertige Phenolverbindungen insbesondere zweiwertige Phenoltypen von der Art der di-substituierten Hydrochinondeivate in Betracht kommen. Weitere Beispiele sind p-Methoxiphenol, 2,5-Di-tert-butyl-p-cresol, Methylhydrochinon und/oder tert.-Butylbrenzkatechin. Der bevorzugte Inhibitor ist das bereits genannte 2,5-Di-tert.-butylhydrochinon. Insbesondere mit diesem Inhibitor gelingt die Herstellung von strahlenhärtbaren polyfunktionellen (Meth)-acrylsäureestern mit hoher Reinheit und insbesondere geringer Eigenfarbe auch unter den vergleichsweise scharfen erfindungsgemäß gewählten Verfahrensbedingungen. Die dabei problemlos anfallenden polyfunktionellen (Meth)acrylsäureester zeichnen sich zudem durch hohe Lagerstabilität aus.

Der Polymerisationsinhibitor bzw. gegebenenfalls das Inhibitorgemisch wird dem Reaktionsgemisch üblicherweise in Mengen von 200 bis 10000 ppm und bevorzugt im Bereich von etwa 300 bis 2000 ppm zugesetzt. Die Zahlenangaben beziehen sich dabei jeweils auf das Gewicht des aus (Meth)acrylsäure und polyfunktionellen Alkoholen bestehenden Reaktionsgemisches.

Als zu veresternde Polyalkohole seien beispielsweise genannt: Ethylenglycol, Propylenglycol, Butandiol-1,4, Hexandiol-1,6, Neopentylglycol, Diethylenglycol, Triethylenglycol, Dimethylolpropan, Glycerin, Trimethylolpropan, Trimethylolhexan, Trimethylolethan, Hexantriol-1,3,5 und Pentraerythrit. Erfindungs gemäß kommen als polyfunktionelle Alkohole insbesondere aber auch die Oxalkylierungsprodukte dieser zuvor genannten polyfunktionellen Alkohole in Betracht, wobei hier den Oxethylierungsprodukten und/oder den Oxpropylierungsprodukten besondere Bedeutung zukommt. Kettenverlängerte polyfunktionelle Alkohole dieser Art können beträchtliche Mengen an Polyalkoxidresten enthalten, beispielsweise 1 bis 50 Mol, vorzugsweise etwa 1 bis 20 Mol Ethylenoxid pro g-Äquivalent an Hydroxylgruppen.

Veresterungskatalysatoren für das erfindungsgemäße Herstellungsverfahren sind handelsübliche organische oder anorganische Säuren oder auch saure Ionenaustauscher, wobei den in der Praxis häufig eingesetzten entsprechenden Verbindungen p-Toluolsulfonsäure und Schwefelsäure besondere Bedeutung zukommt. Die Mengen des Veresterungskatalysators liegen beispielsweise im Bereich von 0,1 bis 5 Gew.-% bezogen auf das Veresterungsgemisch.

Die Umsetzung der Reaktanten wird vorzugsweise bei Sumpftemperaturen von wenigstens etwa 90 °C und insbesondere von wenigstens etwa 100 °C durchgeführt. Besonders geeignet ist der Temperaturbereich bis etwa 150 °C. Dabei kann unter Normaldruck, zweckmäßigerweise aber auch unter abgesenktem Druck gearbeitet werden. Beim Arbeiten mit vermindertem Druck kann in einer besonderen Ausführungsform der Druck in Richtung auf niedrigere Drucke stufenweise oder auch kontinuierlich verringert werden.

Durch die Möglichkeit unter vergleichsweise scharfen Veresterungsbedingungen und gleichzeitig vermindertem Druck zu arbeiten, wird die Reaktionsdauer gegenüber bisher beschriebenen Verfahren stark abgekürzt. So können im erfindungsgemäßen Verfahren Umsatzausbeuten von wenigstens 90 % der Theorie und vorzugsweise von wenigstens etwa 94 % der Theorie im Temperaturbereich von etwa 100 bis 140 °C bei einer Reaktionsdauer von nicht mehr als etwa 10 Stunden und vorzugsweise von nicht mehr als etwa 8 Stunden erzielt werden. Gleichwohl fallen die Reaktionsprodukte als hellfarbige oder durch eine einfache Nachbehandlung wirkungsvoll zu reinigende stabilisierte Masse an. Das den sauren Veresterungskatalysator enthaltende Reaktionsrohprodukt wird einer nachfolgenden Neutralisation unterworfen. Diese Neutralisation kann unter bekannten Naßbedingungen, beispielsweise durch Einsatz von wäßrigen Soda- und gegebenenfalls Natriumchlorid enthaltenden Lösungen erfolgen. In einer bevorzugten Ausführungsform wird allerdings das den sauren Katalysator enthaltende Reaktionsrohprodukt einer trockenen Neutralisation unterworfen. Geeignete trockene Neutralisationsmittel sind die Oxide und/oder Hydroxide der Alkalimetalle, der Erdalkalimetalle und/oder des Aluminiums. Besonders geeignet sind zur Trockenneutralisation entsprechende Verbindungen des Magnesiums bzw. des Calciums.

(Meth)acrylsäure und die Alkohole können für die Veresterung in äquivalenten Mengenverhältnissen eingesetzt werden. Bei den mehr als zweiwertigen Alkoholen ist es allerdings auch ohne weiteres möglich, nur einen Teil der Hydroxylgruppen zu verestern. Zur Vollveresterung kann es zweckmäßig sein, die Säurekomponente in leichtem Überschuß über die zur Veresterung der Hydroxylgruppen erforderliche stöchiometrische Menge einzusetzen. Ein solcher Überschuß kann wenigstens etwa 10 Mol-% ausmachen. Nach Abschluß der Reaktion kann gewünschtenfalls zusätzlich ein Inhibitor dem Reaktionsprodukt beigemischt werden.

Treten bei der Herstellung der Reaktionsprodukte unter den erfindungsgemäßen drastischen Veresterungsbedingungen dann doch einmal leichte Farbverschlechterungen im Reaktionsprodukt auf, so lassen sich diese problemlos durch eine Nachbehandlung mit Entfärbungsmitteln beseitigen. Ein geeignetes Entfärbungsmittel ist beispielsweise Aluminiumoxid.

## Beispiele

### Biespiel 1

In einem 3-Liter-Reaktor wurden 1559,5 g Acrylsäure, 1521,0 g eines ethoxylierten Trimethylolpropans (OH-Zahl: 680 mg KOH/g Substanz), 107,8 g p-Toluolsulfonsäure sowie 4,96 g 2,5-Di-tert.-butylhydrochinons (2000 ppm bezogen auf Produktmenge) eingewogen.

Die Veresterung wurde unter Durchleiten von Luft (40 l/h) und unter Wasserabtrennung durchgeführt. Bei einer maximalen Sumpftemperatur von 105 °C und einem Druck von 400 mbar betrug die Veresterungszeit 6 Stunden.

Rohprodukt:

Säurezahl:      31 mg KOH/g
OH-Zahl:      21,8 mg KOH/g
Gardner Farbzahl:      94,4 %
$H_2O$-Gehalt:0,15 %

Das Rohprodukt wurde durch Zugabe von 103 g $Ca(OH)_2$ und 2-stündigem Rühren bei 80 °C und 50 mbar neutralisiert und anschließend mit Hilfe einer Druckfilternutsche filtriert.

Produkt:

Säurezahl:      < 1 mg KOH/g
OH-Zahl:      24mg KOH/g
Gardner Farbzahl:      < 1

### Beispiel 2

Biespiel 1 wurde mit der Änderung wiederholt, daß die Veresterung bei 120 °C und 700 mbar durchgeführt wurde.

Rohprodukt:

Säurezahl:      20 mg KOH/g
OH-Zahl:      20 mg KOH/g
Umsatz:      95,0 %
Gardner Farbzahl:      3

Das Rohprodukt wurde mit 4 Liter wäßriger 16 Gew.-% NaCl/4 Gew.-% $NaHCO_3$-Lösung gewaschen, mit 200 ppm Hydrochinonmonomethylether nachinhibiert und im Vakuum bei 40 mbar und 80 °C 2 Stunden getrocknet und mit Hilfe einer Druckfilternutsche filtriert.

Produkt:

Säurezahl:      < 1 mg KOH/g
OH-Zahl:      23 mg KOH/g

Gardner Farbzahl:    2 - 3

Beispiel 3

Die Veresterung wurde wie in Beispiel 1 durchgeführt mit der Änderung, daß die Veresterung bei 140 °C und Normaldruck durchgeführt wurde.

Rohprodukt:

Säurezahl:       64 mg KOH/g
OH-Zahl:         29 mg KOH/g
Umsatz:          92,5 %
Gardner Farbzahl:     4

Das Rohprodukt wurde analog zu Beispiel 2 aufgearbeitet.

Produkt:

Säurezahl:       < 1 mg KOH/g
OH-Zahl:         33 mg KOH/g
Gardner Farbzahl:     3-4

Beispiel 4

1320,0 g Acrylsäure, 1861,7 g eines propoxylierten Neopentylglycols (OH-Zahl: 460 mg KOH/g Substanz) sowie 111,4 g p-Toluolsulfonsäure wurden in einen 3-Liter-Reaktor eingewogen und mit 5,37 g 2,5-Di-tert.-butylhydrochinon (2000 ppm bezogen auf Produktmenge) inhibiert. Unter Durchleiten von Luft (40 l/h) wurde die Veresterung unter Wasserabtrennung durchgeführt. Bei einer maximalen Sumpftemperatur von 105 °C bei 400 mbar betrug die Veresterungszeit 6 Stunden.

Rohprodukt:

Säurezahl:       34 mg KOH/g
OH-Zahl:         17 mg KOH/g
Umsatz:          94,4 %   Gardner Farbzahl:    < 1

Das Rohprodukt wurde durch Zugabe von 125 g Ca(OH)$_2$ und 2-stündigem Rühren bei 80 °C und 50 mbar neutralisiert und anschließend mit Hilfe einer Druckfilternutsche filtriert.

Produkt:

Säurezahl:       < 1 mg KOH/g
OH-Zahl:         20 mg KOH/g
Gardner Farbzahl:     < 1

Beispiel 5

368,2 g eines ethoxylierten Trimethylolpropans (OH-Zahl 680 mg KOH/g Substanz), 376,2 g Acrylsäure, 26,5 g p-Toluolsulfonsäure und 0,18 g 2,5-Di-tert.-butylhydrochinon (300 ppm bezogen auf die Produktmenge) wurden in einen 1-Liter-Dreihalskolben eingewogen.

Unter Durchleiten von Luft (20 l/h) wurde die Veresterung unter Wasserabscheidung durchgeführt. Bei einer maximalen Reaktionstemperatur von 105 °C und einem Vakuumsprofil von 2 h/400 mbar, 1 h/270 mbar, 1 h/200 mbar und 2 h/50 mbar betrug die Veresterungszeit 5 Stunden.

Rohprodukt:

Säurezahl:       39,2 mg KOH/g
OH-Zahl:         18 mg KOH/g
Umsatz:          95,4 %
Gardner Farbzahl:     2
H$_2$O-Gehalt:    0,13 %

Das Rohprodukt wurde durch Zugabe von 31,8 g Ca(OH)$_2$ und 2stündigem Rühren bei 80 °C und 50 mbar neutralisiert und anschließend mit Hilfe einer Druckfilternutsche filtriert.

Produkt:

Säurezahl:       < 1 mg KOH/g
OH-Zahl:         21 mg KOH/g
Gardner Farbzahl:     < 1
H$_2$O-Gehalt:    0,17 %

**Ansprüche**

1. Verfahren zur Herstellung von (Meth)acrylsäureestern mehrwertiger Alkohole durch Umsetzung der Reaktanten in Gegenwart von sauren Veresterungskatalysatoren unter Zusatz von alpha-substituierten phenolischen Verbindungen, insbesondere Di-tert.-butylhydrochinon, als Polymerisationsinhibitoren, dadurch gekennzeichnet, daß man mit bei Reaktionstemperatur flüssigen Reaktionsgemischen arbeitet, die wenigstens weitgehend frei von Lösungs- und/oder azeotropen Schleppmitteln sind und dabei das entstehende Kondensationswasser aus der Gasphase des Reaktionsraumes abzieht.

2. Verfahren nach Anspruch 1, dadurch gekennzeichnet, daß man den Reaktionsraum mit einem freien Sauerstoff enthaltenden Gasstrom durchspült.

3. Verfahren nach Ansprüchen 1 und 2, dadurch gekennzeichnet, daß man als Gasstrom Luft oder O$_2$-abgereicherte Gasgemische insbesondere Stickstoff/Luftgemische einsetzt.

4. Verfahren nach Ansprüchen 1 bis 3, dadurch gekennzeichnet, daß die Veresterung bei Sumpftemperaturen von wenigstens etwa 90 °C, vorzugsweise von wenigstens etwa 100 °C und insbesondere im Bereich bis etwa 150 °C durchgeführt wird, wobei bevorzugt wenigstens absatzweise bei vermindertem, gegebenenfalls stufenweise zunehmend vermindertem Druck, gearbeitet wird.

5. Verfahren nach Ansprüchen 1 bis 4, dadurch gekennzeichnet, daß die Inhibitoren in Mengen von 200 bis 10000 ppm, bevorzugt im Bereich von 300 bis 2000 ppm - jeweils bezogen auf das Gewicht des Reaktionsgemisches - eingesetzt werden, wobei das Arbeiten mit unter Reaktionsbedingungen schwer flüchtigen Inhibitoren bevorzugt ist.

6. Verfahren nach Ansprüchen 1 bis 5, dadurch gekennzeichnet, daß die Reaktion auf einen Umsatz von wenigstens 90 % der Theorie, vorzugsweise von wenigstens 94 % der Theorie geführt wird, wobei man bevorzugt im Temperaturbereich von etwa 100 bis 140 °C - gewünschtenfalls unter Vakuum - für eine Reaktionsdauer von nicht mehr als 10 Stunden, insbesondere von nicht mehr als 8 Stunden, arbeitet.

7. Verfahren nach Ansprüchen 1 bis 6, dadurch gekennzeichnet, daß des Reaktionsrohprodukt einer trockenen Neutralisation -bevorzugt mit Oxiden und/oder Hydroxiden der Alkalimetalle, der Erdalkalimetalle und/oder des Aluminiums - unterworfen wird.

8. Verfahren nach Ansprüchen 1 bis 7, dadurch gekennzeichnet, daß die primär anfallenden Reaktionsprodukte einer abschließenden Behandlung mit Entfärbungsmitteln unterworfen werden.

Europäisches
Patentamt

**EUROPÄISCHER RECHERCHENBERICHT**

Nummer der Anmeldung

EP 89 12 3217

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-4 187 383  (F.G. COWHERD)<br>* Spalte 2, Zeile 22 - Spalte 3, Zeile 3; Spalten 7-8; Patentansprüche *<br>--- | 1 | C 07 C  69/54<br>C 07 C  67/08 |
| A | JP-A-5 531 046  (MITUBISHI KASEI KOGYO)<br>* Zusammenfassung *<br>--- | 1 | |
| A | DE-A-2 552 987  (HOECHST)<br>* Seiten 1-3; Patentansprüche; Seite 8, Abschnitt 2 *<br>----- | 1 | |
| | | | **RECHERCHIERTE SACHGEBIETE (Int. Cl.5)**<br><br>C 07 C  69/00<br>C 07 C  67/00 |

Der vorliegende Recherchenbericht wurde für alle Patentansprüche erstellt

| Recherchenort | Abschlußdatum der Recherche | Prüfer |
|---|---|---|
| DEN HAAG | 04-04-1990 | KINZINGER J.M. |

EPO FORM 1503 03.82 (P0403)

**KATEGORIE DER GENANNTEN DOKUMENTE**

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus andern Gründen angeführtes Dokument
.................................................................................
& : Mitglied der gleichen Patentfamilie, übereinstimmendes
   Dokument